Europäisches Patentamt

European Patent Office

Office européen des brevets

(19)

(11) Publication number: **0 077 471**
**B1**

(12) **EUROPEAN PATENT SPECIFICATION**

(45) Date of publication of patent specification: **24.08.88**

(51) Int. Cl.⁴: **A 61 B 6/03**, G 01 T 1/29

(21) Application number: **82108739.2**

(22) Date of filing: **21.09.82**

(54) Computerized tomography apparatus.

(30) Priority: **06.10.81 JP 159120/81**

(43) Date of publication of application:
**27.04.83 Bulletin 83/17**

(45) Publication of the grant of the patent:
**24.08.88 Bulletin 88/34**

(84) Designated Contracting States:
**FR GB NL**

(56) References cited:
**EP-A-0 011 848**
**EP-A-0 037 151**
**DE-A-2 842 787**
**FR-A-2 434 397**

**MEDICAL PROGRESS THROUGH
TECHNOLOGY, Vol. 7, No. 4, 1980, Berlin E.
SCHULTZ et al. "Fan-Beam Computerized
Tomography Systems with Rotating and with
Stationary Detector Arrays ('Third' and 'Fourth'
Generation)" pages 169 to 181**

(73) Proprietor: **KABUSHIKI KAISHA TOSHIBA
72, Horikawa-cho Saiwai-ku
Kawasaki-shi Kanagawa-ken 210 (JP)**

(72) Inventor: **Mori, Issei
151 Mishima Nishinasuno-cho
Nasu-gun Tochigi-ken (JP)**

(74) Representative: **Henkel, Feiler, Hänzel & Partner
Möhlstrasse 37
D-8000 München 80 (DE)**

Courier Press, Leamington Spa, England.

## Description

The present invention relates to a computerized tomography apparatus improved in that reduction of detected radiation data scanned at a high speed is realized by reducing both the number of views and rays.

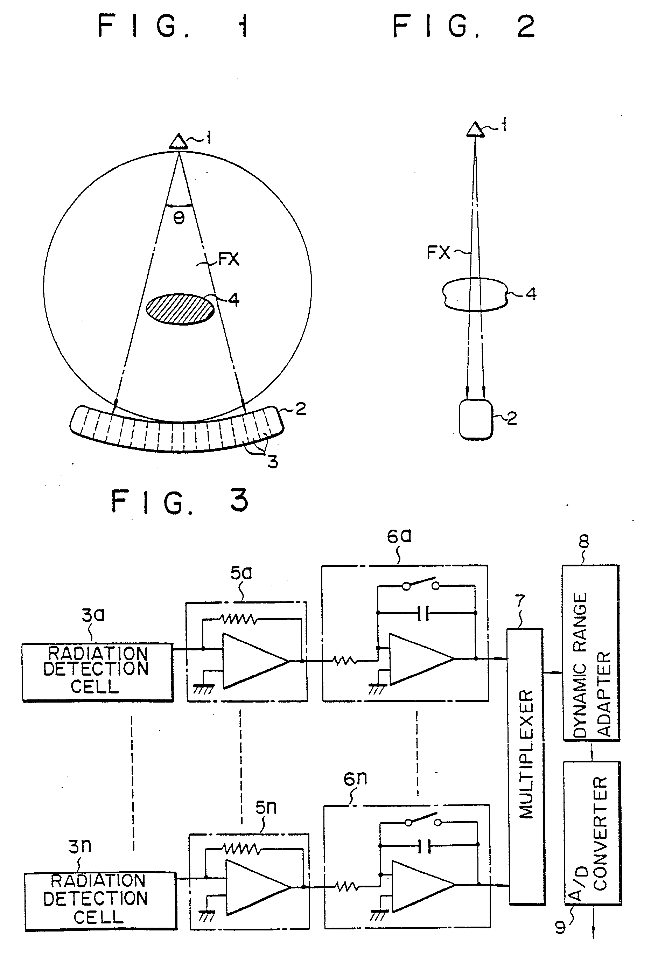

A conventional computerized tomography apparatus (referred to as a CT scanner) is made using an X-ray source 1 for projecting X-ray Fx in the form of a flat fan beam and a multichannel X-rays detector 2 comprising a plurality of radiation detecting elements 3 for detecting said X-rays, said source and detectors being oppositely disposed with an object under diagnosis interposed therebetween, as shown in Figs. 1 and 2. For acquiring X-ray data of the cross sections of the object in every direction, the X-ray source 1 and the X-ray detector 2 are rotated about the object 4 in the same direction and at the same angular speed. After a satisfactory amount of the data is acquired, a digital computer calculates absorption of the penetrating X-rays at individual positions on the cross sections of the object 4. The absorptions of the cross section of the object 4 are used for providing a gradation of a tomogram to be formed. In this way, a clear tomogram is formed of every tissue from soft tissue to hard tissue.

The X-ray detector 2 is made up of a number of radiation detecting cells forming an ionization chamber filled with high pressure gas such as Xe gas or photodiode plus scintillator like a cadmium tungstate scintillation ($CdWO_4$) crystal. The X-ray detector 2 thus constructed detects the energy of the X-ray transmitted through the cross section of the object 4 to provide ionization current as X-ray projection data.

In particular, in acquiring the X-ray projection data, X-ray photons passing through paths connecting the radiation detecting cells 3 of the ionization chamber to the X-ray source 1 (this path is called an "X-ray path") hit the high pressure gas filled in the ionization chamber to ionize the gas. The ionized charge thus produced is extracted. In this way, the energy of the X-ray is detected in the form of ionization current. The ionization current is integrated for a given time, the result of the integration is subjected to A/D conversion. The result of the A/D conversion is the X-ray projection data on the X-ray path. After the data on all the X-ray paths formed at an angular position when the X-ray radiated from the X-ray source transmits through the object, is acquired, the data acquisition on the X-ray paths formed at the next angular position is performed. Similarly, the data acquisition is performed at the subsequent angular positions.

For signal processing on the data acquisition, as shown in Fig. 3, a number of radiation detecting cells 3a to 3n are respectively coupled with amplifiers 5a to 5n for amplifying the output signals from the detecting cells 3a to 3n and for applying them to integrators 6a to 6n. X-ray photons arriving at the detecting cells 3a to 3n are detected by these cells and amplified by the amplifiers 5a to 5n to proper values, and then integrated by integrators 6a to 6n, respectively. The output signals from the integrators 6a to 6n are selectively extracted in a sequential manner and are applied to a dynamic range adaptor 8 for dynamic range adjustment. The dynamic range adjusted signal is then applied to an A/D converter 9 where it is converted into a digital signal. Thus digitized X-ray projection data for each channel are applied to a host system such as digital computer. Incidentally, the dynamic range adaptor is not essential to this signal processing system of the CT scanner under discussion. This CT scanner is called a third generation CT scanner. In addition to the third generation CT scanner, there are first, second and fourth generation CT scanners, although not shown. In first generation CT scanner, an X-ray source for radiating X-rays in pencil beam fashion and an X-ray detector using a single radiation sensor are oppositely disposed. In operation, the combination of the detecting cell and the X-ray source are translated. After one translation is completed, the X-ray projection angle is changed with respect to the object, or rotated about the object, and then the translation of the cell and X-ray combination is repeated. The first generation CT scanner is of translate/rotate type. In the second generation CT scanner, an X-ray source for projecting a relatively narrow X-ray beam in a fan shape is used in place of the pencil beam radiation X-ray source of the first generation CT scanner. The X-ray detector uses a plurality of radiation sensing cells for covering the width of the radiated fan beam. The scanning operation by the CT scanner is similar to that of the first generation one. In the fourth generation CT scanner, a number of radiation sensing cells are disposed in a circle. In taking tomograms of the object or acquiring X-ray projection data, the X-ray source is rotatably moved radiating the fan X-ray beam, while the detector is stationary.

The resolution of a reproduced picture depends on how fine the picture elements are. However, improvement of the resolution of the reproduced picture cannot be expected unless the pitch for sampling the X-ray projection data is also fine.

To obtain a CT scanner with high resolution, the number of channels of the X-ray detector and of the data acquiring system must be increased.

The same thing is not applied for the first and second generation CT scanners because these scanners are accompanied by a translation movement. In these CT scanners, however, the data acquiring speed is quite slow and the CT scanners have a problem of long data acquiring time.

It is safe to say that the third and fourth CT scanners with high resolution have a large number of channels.

The reason for this is as follows. In the third generation CT scanner, the scanning depends only on the rotational movement. Further, a relationship between the number of the radiation detecting cells 3 and an angular scope of the fan beam is fixed and it is impossible to change the sampling pitch for the object. Therefore, for making the sampling pitch finer, the pitch of the channels must be made finer, or the number of the detecting cells must be increased.

In the fourth generation CT scanner, the X-ray detecting elements 3 are circularly arranged and only the X-ray source is rotated. The sampling pitch is controllable by controlling the rotation angle for each sampling. Even if the sampling pitch is made fine to increase the number of views, a spatial frequency response can be extended up to high frequencies if the width of an opening the radiation detecting cell is wide. In this respect, the size of the opening of the detecting cell must be small. For covering the expanded width of the fan beam due to the discrete arrangement of the tiny radiation detecting cells, the number of the channels is inevitably increased.

In a high resolution CT scanner, the number of channels and the views (projections) as well are large. As is well known, to reproduce exactly the high spatial frequencies of the object, the number of the views must be large. Typical examples of the relationship of the number of channels to the number of views at the present time are:

Third generation:
515 channels, 400 views/4.5 sec. scan or 600 views/9 sec. scan

Fourth generation:
1088 channels, 1088 views/3 sec. scan, 124 rays/view

The amount of data acquired by the fourth generation CT scanner is generally larger than that of the third generation. In the above example, the total number of data is $1.3 \times 10^6$, with the time for each data (one ray) as 2.2 μs.

In the fourth generation CT scanner, a cutoff frequency of MFT (modulation transfer function) which means a high spatial frequency of the reproduced picture to an original picture is approximately 8 lp (line pair)/cm which means that a spatial frequency is eight pairs of black and white bars per 1 cm. When it is desired to expand the cutoff frequency to 40 lp/cm, for example, which is five times the above, that is, the cutoff frequency of the general tomographing, it is necessary that the number of channels of the X-ray detector must be about five times, or 5440, and the number of rays is also about five times, or 6,205 rays/view.

As a result, a total number of data is $3.38 \times 10^7$. With such an amount of data, it is very difficult to apply high speed scanning for that data acquisition, that is, to rapidly construct an image. Nevertheless, there is still strong demand for shortening the scanning time and improving artifacts of image.

The prior art CT scanner has a plurality of operation modes for dealing with this problem. When high speed scanning is required, the number of data is reduced to speed up the acquisition of data. When no high speed scanning is required, a sufficient number of data is acquired to improve the picture quality.

For reducing the data in a high speed scanning mode, the third generation CT scanner has reduced the number of views or the number of projections, and the fourth generation CT scanner has reduced the number of rays per view, that is, the number of X-ray paths per view.

Both the third and fourth generation CT scanners sample the object under diagnosis every time that the X-ray source rotates at a fixed angle. Accordingly, the reduction of the data may be attained by controlling the rotating angle pitch for projecting.

The reduction of the number of views or rays leads to a deterioration in picture quality.

When the number of the views is reduced, streak artifacts occurs. For avoiding these artifacts, a band of the convoluton filtering process is narrowed to reduce a spatial resolution.

The reduction of the rays results in an increase of angle between the sampling pitches. This is clearly related to a deterioration of the spatial resolution.

A moving portion or a portion difficult to keep in a motionless state for a long time, must be tomographed at a high speed, even though the spatial resolution is somewhat impaired.

The views and the rays are not parameters designed independently. These parameters are given depending on the desired spatial frequency band. For an optimum design, if one of the parameters is reduced, the other must also be reduced; otherwise, data larger than the data necessary for securing a given picture quality is collected, with the result that time is consumed in unnecessary data acquiring and processing time. Although such is the case, the prior CT scanner controls only one of the parameters, as disclosed in U.S. (Patent No. 4,075,492).

Further, in "Medical Progress Through Technology", vol. 7, No. 4, 1980, pages 169—181, are described fan beam computerized tomography systems with rotating and with stationary detector arrays, i.e. tomography systems of the third and fourth generation systems, which can, optionally, connect neighbouring detectors in order to increase density resolution, however, at the cost of spatial resolution.

Let us consider at what speed the prior art CT scanner may operate when the data of $3.38 \times 10^7$ is necessary for attaining the cutoff frequency 40 lp/cm, as mentioned above. Even if the sampling pitch is made two times larger than the normal sampling pitch, the assumption is that sacrifice in resolution in high speed scanning is unavoidable and the data will reach even $1.69 \times 10^7$. In an acquistion time system two bytes of memory capacity for each data are required. For storing such an amount of data, 12 megabytes (MB) of memory capacity is necessary.

For a scanning speed of 0.5 sec. at the ordinary sampling pitch, a storing medium which allows data to be written therein at 67.6 MB/sec. is needed.

At the present stage of technology, a magnetic disc is most suitable for the data storing medium for storing a large amount of data. A magnetic disc operable at such a high speed is very expensive and also requires an A/D converter and a data transfer system, which are operable at a corresponding high speed. Therefore, time taken for data acquisition and processing must be shortened such that the number of both the views and the rays are reduced and the data are reduced in a well balanced manner. With a shortening in the data acquisition and processing time, the data storing requirement is eased reducing the cost.

The third and the fourth generation CT scanners will be further described.

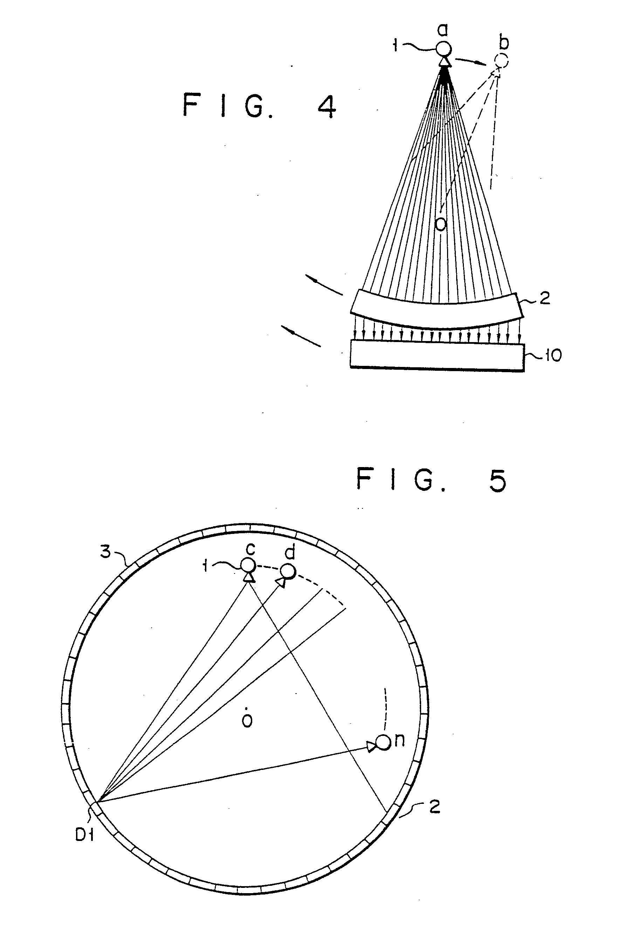

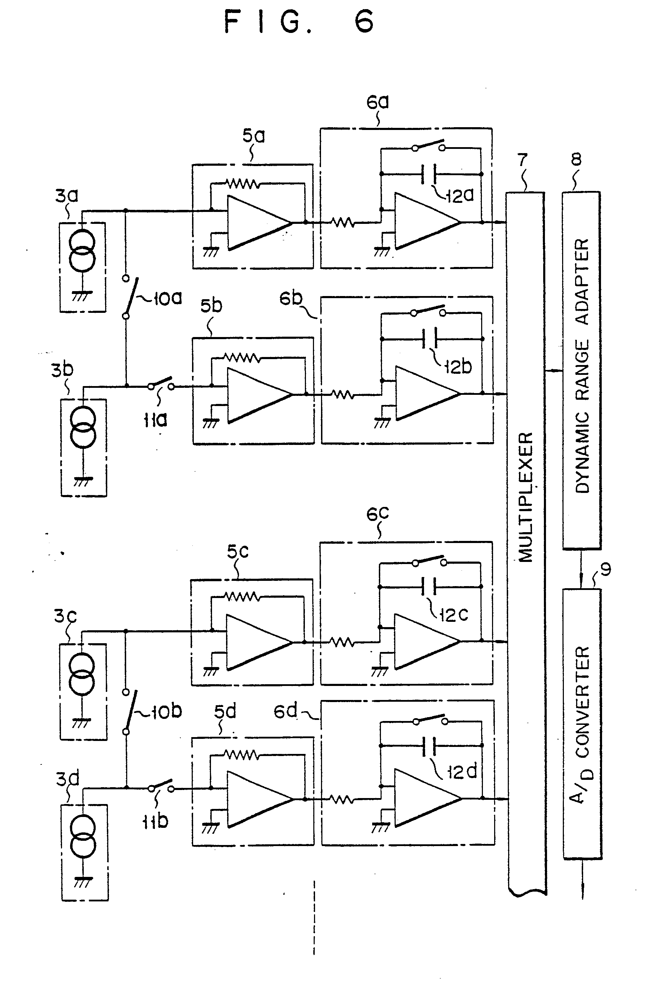

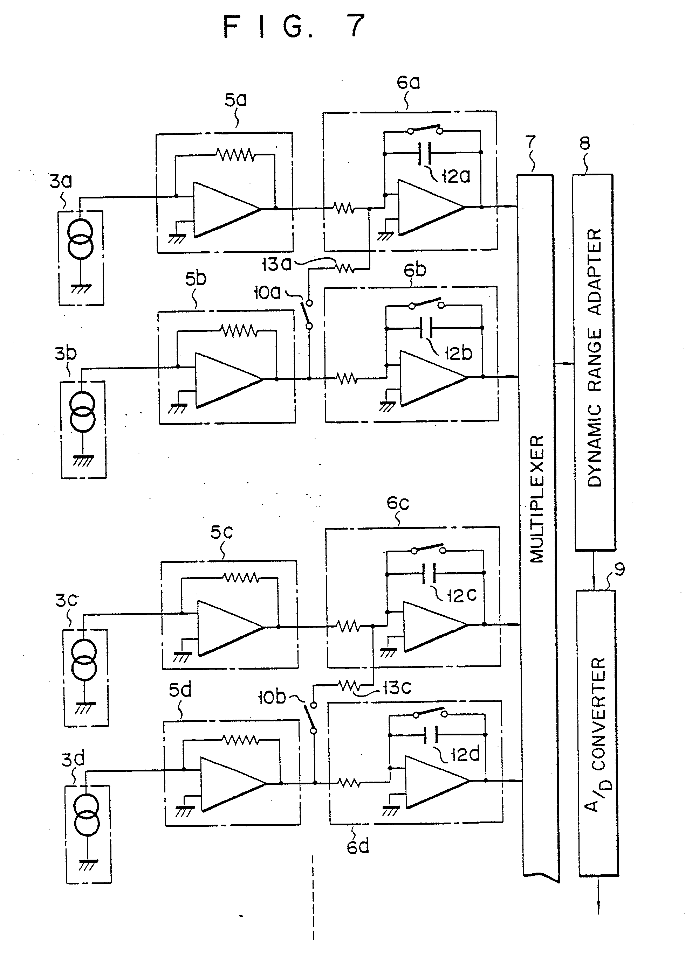

Fig. 4 illustrates a third generation CT scanner. As shown, an X-ray source 1 and an X-ray detector 2 are concurrently rotated about a center 0 in a field or data acquiring area. An X-ray radiation of one pulse is performed at a position *a* of the X-ray source 1 to first acquire the data and then data acquisition of the first view is completed. In this case, the number of the rays is equal to that of the channels. The data acquisition of the second view is performed at an X-ray source position *b*. In a high speed scanning mode, the rotation angle /aob of one angular shift is larger than the usual one. That is, the number of views in one rotation of the X-ray source is reduced. In this way, the CT scanner copes with the high speed scanning operation.

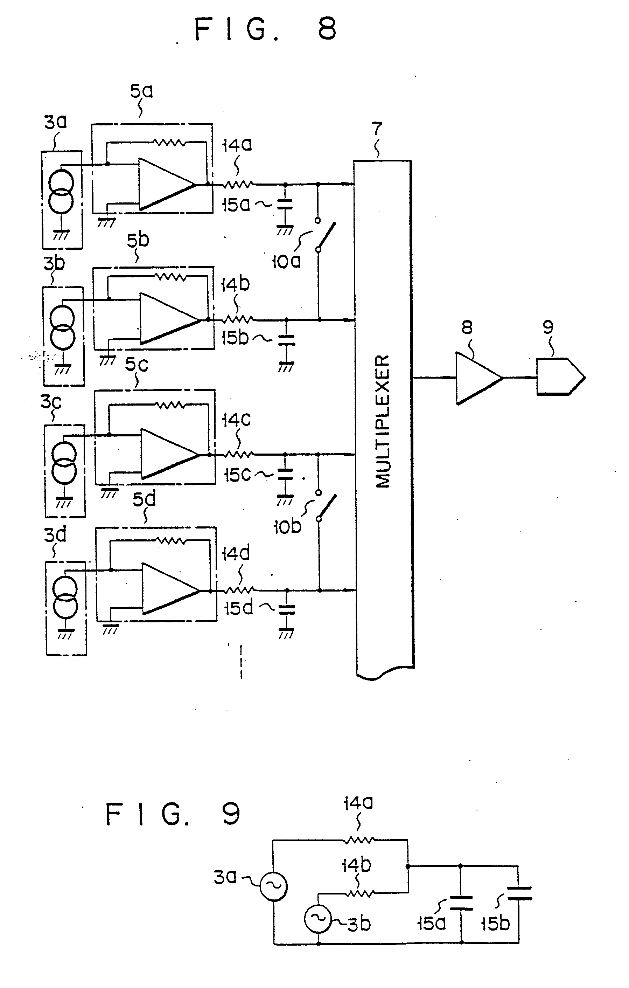

Fig. 5 shows a fourth generation CT scanner. As shown, an X-ray source 1 rotates about the center 0 of a tomograph object, radiating the X-ray fan beam toward the center, a circular detector is motionlessly mounted with just the source rotating. When the X-ray source 1 is located at a position *c*, the data of the first ray is acquired by a group of radiation detecting cells located within a range of the fan X-ray beam radiated therefrom. Then, the X-ray source 1 moves to a position *d*. Here, the data on the second ray is acquired by a group of radiation detecting cells located within a range of the fan beam of X-ray radiated therefrom. This data acquisition is repeated up to a position *n*. When the data acquisition is completed at the position *n*, one view as seen from the D1 radiation detecting cell is completed. Views of the other radiation cells are similarly obtained. In a high speed scanning mode, the rotation angle /cDld is made large. In this case, only the number of rays is reduced, while the number of cells is fixed. Therefore, the total number of views for all the cells is unchanged.

For reducing the number of both rays and views, the prior scanner needs an additional circuit. Accordingly, the prior scanner reduces either the number of rays or views in the high scanning speed mode not only from a cost/performance standpoint but also because the extremely high-speed scanning and extremely high resolution are not intended. The rays and the views must be reduced in a well balanced manner. If only one of the views or the rays is reduced, the data amount is not reduced much, and more adversely superfluous data is included. Accordingly, the prior art CT scanner involves many problems in data write speed required for the data storing medium, the resulting operation speed of the A/D converter and the data transfer system, the data processing speed, etc.

Accordingly, an object of the present invention is to provide a computerized tomography apparatus which secures an ordinary high resolution and realizes a reduction of the detected radiation data by reducing the number of both the views and rays.

In order to fulfil the said object there is provided a computerized tomography apparatus comprising: a radiation source for projecting a fan-shaped radiation beam toward an object to be examined, said radiation source being rotatably arranged relative to the object so as to be movable about a rotation axis through a first rotation angle or a second rotation angle, said first rotation angle being smaller than said second rotation angle; detector means positioned to detect the radiation emanating from said radiation source and passing through the object, said detector means having a plurality of radiation sensing cells aligned to produce first signals from the radiation impinging thereon; and means for optionally combining the first signals from adjacent cells, characterized in that said combining means generate second signals by summation of said first signals supplied from at least every two adjacent cells when said radiation source projects the fan-shaped radiation beam with the radiation source being rotated through the second rotation angle.

With such an arrangement, the computerized tomography apparatus according to the present invention provides a high-resolution reproduced picture at a low speed or normal scanning mode and reduces the amount of the radiation detected data by reducing the number of both the views and the rays. Accordingly, the requirement for the data transfer time is relaxed and the requirement for a data writing speed to a medium for storing data is remarkably relaxed. A necessary number of A/D converters for A/D converting a radiation dose output from each channel, which is determined by the operation speed of the scanner in a high speed operation mode, is reduced to a value corresponding to the reduced number of the channels, resulting in reduction of cost. With the data fetching capability of the system fixed, a high scanning speed can be expected in comparison with the case where the system has not means for composing the outputs for a plurality of detectors.

This invention can be more fully understood from the following detailed description when taken in conjunction with the accompanying drawings, in which:

Fig. 1 is a schematic illustration of a third generation CT scanner;

Fig. 2 is a schematic side view of an object under diagnosis and the radiation detector shown in Fig. 1;

Fig. 3 is a circuit diagram of a prior art data acquiring system for acquiring detected radiation dose data with a plurality of channels;

Fig. 4 are schematic illustrations for explaining the data acquiring operation by a third generation CT scanner;

Fig. 5 shows a schematic illustration of an operation for acquiring detected radiation dose data by a fourth generation CT scanner;

Figs. 6 to 8 illustrate circuit arrangements of a data acquiring system for acquiring detected radiation dose data with a plurality of channels, which are different embodiments of the invention;

Fig. 9 is an equivalent circuit diagram of two channels coupled with each other in the circuit shown in Fig. 8;

Figs. 10 and 11 show circuit arrangements of data acquiring system for acquiring detected radiation dose data with a plurality of channels which are other embodiments of the present invention.

Preferred embodiments of the present invention will be described referring to Figs. 6 to 11.

Fig. 6 shows a circuit diagram of a data collecting system which is an embodiment of the present invention. In Fig. 2, like reference numerals are used for designating like portions and no explanation thereof will be given here. Basically, the circuit arrangement of Fig. 6 is the same as that of Fig. 3. For ease of illustration, the 1st to 4th channels are illustrated and since the output impedance of the radiation sensing cells 3a to 3d are high, these may be expressed as a constant current signal source. Analog switches 10a and 10b are proved which respectively connect the outputs of two pairs of the adjacent radiation sensing cells 3a and 3b of the first and second channels and the radiation sensing cells 3c and 3d of the third and fourth channels in the data sensing system. Analog switches 11a and 11b are connected in series in the input circuits of amplifiers 5b and 5d, respectively. When the switches 11a and 11b are closed but the switches 10a and 10b are opened, the detected signals of the two adjacent channels are composited.

Namely, in a low speed or normal scanning mode in which a sufficient number of data are obtained to improve a resolution, a controller (not shown) opens the analog switches 11a and 11b but closes the analog switches 10a and 10b. Accordingly, since the channels are independently operable, the data of all the channels may be collected as in the prior art.

In a high speed scanning mode for reducing the number of the data, the controller closes the analog switches 10a and 10b and opens the analog switches 11a and 11b. As a result, the two pairs of output signals from the radiation detecting cells 3a and 3b and 3c and 3d, each pair of the output signals being produced from two adjacent channels are respectively applied to the amplifiers 5a to 5c, respectively. Thus, the output signals from the two adjacent channels are combined together. In this case, the controller controls the multiplexer 7 by incrementing it every two channels. That is, the output signals from the integrators are progressively selected in a skipping way in the order of the output signal from the integrator in the first channel, the output of the integrator 6c in the third channel, the output of the integrator in the fifth channel, ... . Since the outputs of the radiation detecting cells 3a to 3d have sufficiently high impedance, the output signals from the two radiation detecting cells are composed and the composite one is applied to the single amplifier 6a or 6b.

In this way, the effective number of the radiation detecting cells are reduced to half by controlling only the analog switches 10a, 11a, 10b, 11b, and the multiplexer 7. Accordingly, the rotation angle of the X-ray source 1 for each sampling is allowed to be large. Further, in the third generation the views and the rays as well may be reduced and in the fourth generation the number of the views and the rays as well may be reduced. In this way, the number of data may be held down to a proper value.

A circuit arrangement of Fig. 7 is also so designed that the detected output signals of the two channels are composed together, as in the previous case.

In this circuit, the two adjacent channels are treated as a pair unit. In the first pair of channels a series circuit including an analog switch 10a and a resistor 13a is connected between the input of an integrator capacitor 12a of an integrator circuit 6a in one channel and the output of an amplifier 5b in the other channel. In the second pair of channels, a series circuit including an analog switch 10b and a resistor 13c is likewise connected between the input of an integration capacitor 12c of an integration circuit 6c in one channel and the output of an amplifier 5d in the other channel. Resistance of the resistors 13a and 13c is equal to that of the integration resistors of the integration circuits 6a and 6c.

When the analog switch 10a is closed, the output signal from the amplifier 5b in the other channel is applied through the resistor 13a to the input of an operational amplifier of the integrator 6a of one channel. Similarly, when the analog switch 10b is closed, the output signal from the amplifier 5d in the other channel is applied through the resistor 13c to the input of an operational amplifier in one channel.

Accordingly, if the output impedance of the amplifiers is sufficiently low, the integration capacitor 12a (or 12b) or the integration circuit 6a (or 6c) is charged proportional to the sum of the detected output signals from the radiation sensing cells 3a and 3b (or 3c and 3d) in the first (or second pair) of channels, to produce an integrated value proportional to the sum of the output signals for the corresponding pair of channels. Accordingly, if the muliplexer 7 is incremented every two channels by the controller, much the same operation as that of Fig. 6 may be attained. Thus, the normal scanning mode and the high speed scanning mode may be performed by properly controlling the analog switches 10a and 10b and the multiplexer 7.

Since an amplifier with a sufficiently low output impedance is available, the present embodiment may easily be realized.

Turning now to Fig. 8, there is shown another embodiment of the present invention, which has a somewhat different construction from that of the above two embodiments. In the channels, amplifiers 5a to 5d have at their poststage series circuits which include resistors 14a to 14d of resistance Rb and capacitors 15a to 15d of capacitance Ca, respectively. The capacitors 15a to 15d are each grounded at one end. The output signals at the junction points between the resistors 14a to 14d and the capacitors 15a and 15d are applied to a multiplexer 7. Junction points between the resistors 14 (14a to 14d) and the capacitors 15 (15a to 15d) in the two adjacent channels are connected by a switch 10 (10a to 10b). For example, in the first pair of the two adjacent channels including the sensing cells 3a and 3b, junction points between the resistor 14a and the capacitor 15a and between the resistor 14b and the capacitor 15b are connected by an analog switch 10a. In the second pair of the channels which includes the sensors 3c and 3d, an analog switch 10b connects the junction points between the resistor 14c and the capacitor 15c and between the resistor 14d and the capacitor 15d. If the output signal of the CR circuit which includes the resistor 14 and the capacitor 15 is read out at sampling periods approximate to (from a fraction to a multiple) a time constant τ as the product of the resistance Rb of the resistor 14 and the capacitance Ca of the capacitor C15, the CR circuit functions like an integrator circuit. The electrical performance of the data collecting system of the present embodiment is inferior to that of the above-mentioned embodiments, but its economical feature is superior.

In a normal scanning mode, the analog switches 10a and 10b are open and the multiplexer 7 is incremented in a normal manner.

In a high speed scanning mode, the analog switches 10a and 10b are closed and a controller (not shown) controls the multiplexer 7 so as to sequentially select the output signals from the channels every two channels. The detected output signals are applied to the corresponding time constant circuits each made up of the resistor 14 (14a to 14d) of the resistance Rb and the capacitor 15 (15a to 15d of the capacitance Ca, thereby to charge the capacitor 15 up to a potential corresponding to the detected output level. The output signals from the time constant circuits of the two pairs of channels are applied to the multiplexer 7 when the corresponding analog switches 10a and 10b are closed. An equivalent circuit of the time constant circuits of the first pair of the channels is as shown in Fig. 9. Also in this case, the time constant τ is given by $\tau = (Rb/2) \times 2ca = Ca \times Rb$, and is equal to that when the analog switch is open. Accordingly, when in a normal scanning mode, the charged level of each channel is read out every 1 ms, the additive value of the charged levels of the two channels may be obtained every 1 ms also in a high speed scanning mode if the output signals from the time constant circuits are selected every two channels and read out at 1 ms periods.

Let us return to the CT scanner with the abovementioned data collecting system for collecting the detected data of radiation dose shown in Fig. 6.

In the fourth generation CT scanner, for obtaining a resolution of 40 lp/cm, for example, data of $3.38 \times 10^7$ is required.

To obtain this data for three seconds, a writing speed required for storing the data into a memory with a large memory capacity is 33.8 Mega words/sec or 67.6 Mega bytes/sec.

Normally, one word of the data treated in the CT scanner is formed of two bytes.

The CT scanner involves problems also of the writing speed of data into a data storing medium and the A/D converter for converting the analog detected data into digital data.

The A/D converter with a sufficient number of bits for the CT scanning (16 bits because one word is formed of 2 bytes) is generally expensive and does not have high operation speed. In a typical example, if about 20 μs is taken for processing one data, for processing the data of $3.38 \times 10^7$ for one second, the necessary number of A/D converters is

$$676 (3.38 \times 10^7 \times 2 \times 10^{-5} \times 1 = 676).$$

In this case, even if the resolution is reduced by half unwillingly, the number of channels is unchanged and therefore the number of the A/D converters required is also unchanged. The writing speed of the data storing medium is 33.8 Mega bytes, as mentioned above.

In the data acquisition system designed such that the sampling time is doubled and the data of two channels are combined together into one data in a high speed scanning mode, since the number of channels used is halved, the necessary number of the A/D converters is halved to only 338, and the total number of data is 1/4 compared with the acquisition system that the channel data are not combined and the sampling pitch is not large. Therefore, the data writing speed is only 16.7 Mega bytes. In the case of an apparatus capable of obtaining 40 lp/cm for one second scanning, the scanning time is half, 0.5 sec. if it is merely employed to double the sampling pitch. In this case, however, if two channel data are combined together, the scanning time is halved again to 0.25 sec.

According to the present invention, the following useful effects are attained;

(1) In a high speed scanning mode, the number of views (or rays) is reduced with some sacrifice of the picture quality as given by the resolution and artifacts, and the number of rays (or views) is correspondingly reduced. With this feature, the scanning time is remarkably reduced with little deterioration of the picture quality.

(2) Since the above feature reduces the number

of data, the memory capacity of the data storing medium is also saved, leading to shortening of picture reproducing time.

While the above description has been made on the fourth generation CT scanner, the same thing applies to the third generation CT scanner. Hence, no further description of it will be given to avoid duplication.

The number of the combined channels is not limited to two, but may be any other suitable number such as three or four. Further, the radiation used may be γ rays or any other rays, in addition to the X-rays.

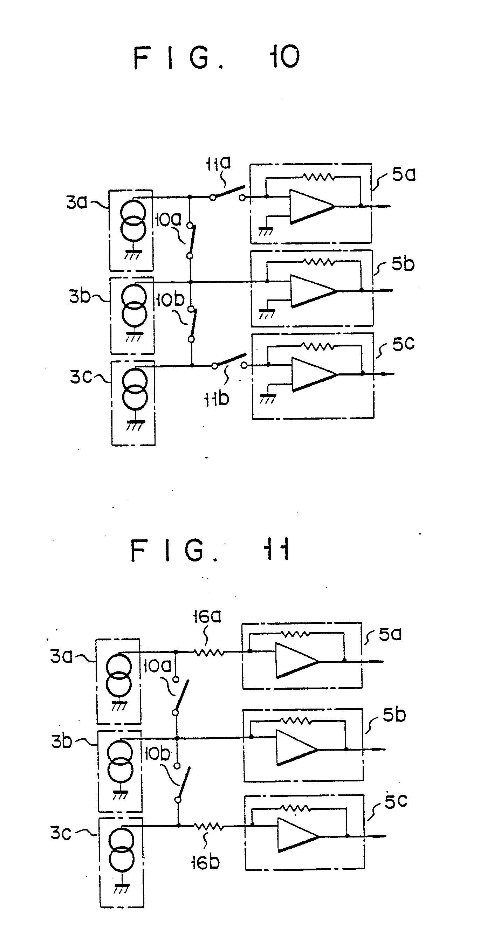

Circuit arrangements of the data collecting system for combining the three channels data together are illustrated in Figs. 10 and 11. In the embodiment shown in Fig. 10, an analog switch 10a is connected between the output terminals of the radiation detecting cells 3a and 3b, and another analog switch 10b is inserted between the sensing cells 3b and 3c. Analog switches 11a and 11b are connected in series between the ouput terminals of the detecting cells 3a and 3c and the input terminals of amplifiers 5a and 5c, respectively, as shown. In a normal scanning mode, the analog switches 10a and 10b are opened while the analog switches 11a and 11b are closed. In a high speed scanning mode, the analog switches 10a and 10b are closed while the analog switches 11a and 11b are opened. With the circuitry thus formed by the switches, the output signals from the three detecting cells 3a to 3c are applied to the single amplifier 5b, thereby obtaining the composite value of these three detected output signals. Although not shown, the multiplexer 7 is of course so controlled as to select the output signals every three channels.

In the case of Fig. 11, the resistors 16a and 16b are used in place of the analog switches 11a and 11b in Fig. 10. This circuit arrangement allows the detected output signals from the detecting cells 3a to 3c to be combined and applied to the input of a center amplifier 5b with no resistor.

## Claims

1. A computerized tomography apparatus comprising:

a radiation source (1) for projecting a fan-shaped radiation beam (FX) toward an object (4) to be examined, said radiation source being rotatably arranged relative to the object so as to be movable about a rotation axis (0) through a first rotation angle or a second rotation angle, said first rotation angle being smaller than said second rotation angle;

detector means (2) positioned to detect the radiation emanating from said radiation source and passing through the object, said detector means having a plurality of radiation sensing cells (3) aligned to produce first signals from the radiation impinging thereon; and

means for optionally combining the first signals from adjacent cells,

characterized in that said combining means (10a, 11a, 10b, 11b; 13a, 13c; 14a—d, 15a—d) generate second signals by summation of said first signals supplied from at least every two adjacent cells (3a, 3b; 3c, 3d) when said radiation source projects the fan-shaped radiation beam with the radiation source being rotated through the second rotation angle.

2. A computerized tomography apparatus according to claim 1, wherein said means for generating said second signals includes switching means (10a, 10b, 11a, 11b).

3. A computerized tomography apparatus according to claim 2, wherein said switching means is at least one analog switch connected between a channel including one of said radiation detector cells and a radiation dose calculating circuit (3a, 5a) and at least another adjacent channel (3b, 5b; 3c, 5c; 3d, 5d) with such a construction, and is caused to close to combine the radiation dose sensed by at least two of said radiation detector cells.

4. A computerized tomography apparatus according to claim 2, wherein said switching means is at least one analog switch connected between adjacent channels so that when closed, said analog switch permit combining a radiation dose obtained by a radiation dose calculating means in one channel and a radiation dose obtained by a radiation dose calculating means in at least one other channel when said radiation source is rotated through said second rotation angle.

## Patentansprüche

1. Computertomographiegerät mit
einer Strahlenquelle (1) zur Projektion eines fächerförmigen Strahlungsbündels (FX) auf ein zu untersuchendes Objekt (4), wobei die Strahlenquelle bezüglich des Objekts drehbar angeordnet ist, so daß sie um eine Rotationsachse (0) um einen ersten Drehwinkel oder einen zweiten Drehwinkel bewegbar ist, wobei der erste Drehwinkel kleiner ist als der zweite Drehwinkel;

einer Detektoreinrichtung (2), die so angeordnet ist, daß sie die von der Strahlenquelle ausgesandten und durch das Objekt hindurchgegangenen Strahlen aufnimmt, wobei die Detektoreinrichtung eine Anzahl von in einer Reihe ausgerichteter, strahlungsempfindlicher Zellen aufweist, zur Erzeugung erster Signale gemäß der auf sie auftreffenden Strahlung; und

Einrichtungen zum beliebigen Mischen der ersten Signale benachbarter Zellen,

dadurch gekennzeichnet, daß die Mischeinrichtungen (10a, 11a, 10b, 11b; 13a, 13c; 14a—d, 15a—d), durch Summierung der ersten von mindestens zwei benachbarten Zellen (3a, 3b; 3c 3d) erzeugten Signale zweite Signale erzeugen, wenn die Strahlenquelle das fächerförmige Strahlenbündel erzeugt, während die Strahlenquelle durch den zweiten Drehwinkel gedreht wird.

2. Computertomographiegerät nach Anspruch 1, dadurch gekennzeichnet, daß die Einrichtungen zur Erzeugung der zweiten Signale Schalteinrichtungen (10a, 10b, 11a, 11b) einschließen.

3. Computertomographiegerät nach Anspruch 2, dadurch gekennzeichnet, daß die Schalteinrichtung aus mindestens einem Analogschalter besteht, der zwischen einen Kanal, der mindestens eine der Strahlendetektorzellen und einen Strahlendosis-Berechnungskreis (3a, 5a) einschließt, und wenigstens einen weiteren Kanal (3b, 5b; 3c, 5c, 3d, 5d) gleicher Art geschaltet ist und geschlossen wird, um die Strahlendosis die von wenigstens zwei der Strahlendetektorzellen registriert wurde, zu mischen.

4. Computertomographiegerät nach Anspruch 2, dadurch gekennzeichnet, daß die Schalteinrichtung aus mindestens einem Analogschalter besteht, der mit benachbarten Kanälen verbunden ist, so daß der Analogschalter nach dem Schließen die Mischung einer Strahlungsdosis, die von einer Strahlendosis-Berechnungseinrichtung in einem Kanal und einer Strahlendosis, die durch eine Strahlendosis-Berechnungseinrichtung in wenigstens einem anderen Kanal erhalten werden, ermöglicht, wenn die Strahlenquelle durch den zweiten Drehwinkel gedreht wird.

**Revendications**

1. Un appareil de tomographie assisté par ordinateur, comprenant:

—une source de rayonnement (1) pour projeter un faisceau de rayonnement (FX) en forme d'éventail en direction d'un objet (4) à examiner, ladite source de rayonnement étant disposée de façon tournante par rapport à l'objet pour être déplaçable autour d'un axe de rotation (O) selon un premier angle de rotation ou un second angle de rotation, ledit premier angle de rotation étant plus petit que ledit second angle de rotation;

—un moyen détecteur (2) positionné de façon à détecter le rayonnement provenant de ladite source de rayonnement et passant au travers de l'objet, ledit moyen détecteur comportant une pluralité de cellules de détection de rayonnement (3) alignées de façon à produire des premiers signaux en fonction du rayonnement arrivant sur elles; et

—des moyens pour combiner facultativement les premiers signaux provenant de cellules adjacentes,

caractérisé en ce que lesdits moyens de combinaison (10a, 11a, 10b, 11b; 13a, 13c; 14a—d, 15a—d) produisent des seconds signaux par sommation desdits premiers signaux fournis par au moins chaque couple de cellules adjacentes (3a, 3b; 3c, 3d) lorsque ladite source de rayonnement projette ledit faisceau de rayonnement en forme d'éventail, la source de rayonnement étant entraînée en rotation selon le second angle de rotation.

2. Un appareil de tomographie assisté par ordinateur selon la revendication 1, dans lequel lesdits moyens de génération desdits seconds signaux comprennent des moyens de commutation (10a, 10b, 11a, 11b).

3. Un appareil de tomographie assisté par ordinateur selon la revendication 2, dans lequel lesdits moyens de commutation sont constitués par au moins un commutateur analogique connecté entre un canal contenant une desdites cellules de détection de rayonnement et un circuit de calcul de dose de rayonnement (3a, 5a), et au moins un autre canal adjacent (3b, 5b; 3c, 5c; 3d, 5d) ayant une telle structure, et qui est amené en condition de fermeture pour combiner la dose de rayonnement détectée par au moins deux desdites cellules de détection de rayonnement.

4. Un appareil de tomographie assisté par ordinateur selon la revendication 2, caractérisé en ce que lesdits moyens de commutation sont constitués par au moins un commutateur analogique connecté entre des canaux adjacents de telle sorte que, ledit commutateur analogique, lorsqu'il est fermé, permette la combinaison d'une dose de rayonnement obtenue par un moyen de calcul de dose de rayonnement dans un canal et une dose de rayonnement obtenue par un moyen de calcul de dose de rayonnement dans au moins un autre canal lorsque ladite source de rayonnement est entraînée en rotation selon ledit second angle de rotation.

# FIG. 1

# FIG. 2

# FIG. 3

RADIATION DETECTION CELL

3a

5a

6a

8

RADIATION DETECTION CELL

3n

5n

6n

7

MULTIPLEXER

DYNAMIC RANGE ADAPTER

A/D CONVERTER

9

F I G. 4

F I G. 5

F I G. 6

0 077 471

3

# F I G. 7

# FIG. 8

# FIG. 9

5

# F I G .  10

# F I G .  11